# EUROPÄISCHE PATENTSCHRIFT

(11) **EP 2 287 164 B1**
(45) Veröffentlichungstag und Bekanntmachung des Hinweises auf die Patenterteilung: **22.01.2014**
(21) Anmeldenummer: 10181043.0
(22) Anmeldetag: 02.11.2005
(51) Int. Cl.: C07D 473/04, A61K 31/522

(54) **Verfahren zur Herstellung chiraler 8-(3-amino-piperidin-1yl)-Xanthine**
Process for the manufacture of chiral 8-(3-aminopiperidin-1-yl)-xanthines
Procédé pour la préparation des 8-(3-aminopiperidin-1-yl)-xanthines chirales

(30) Priorität: 05.11.2004 DE 102004054054
(43) Veröffentlichungstag der Anmeldung: 23.02.2011
(62) Teilanmeldung aus: 05804601.2
(73) Patentinhaber: BOEHRINGER INGELHEIM INTERNATIONAL GMBH, 55216 Ingelheim am Rhein (DE); Boehringer Ingelheim Pharma GmbH & Co. KG, 55216 Ingelheim am Rhein (DE)
(72) Erfinder: Pfrengle, Waldemar, 88400, Biberach (DE); Pachur, Thorsten, 88400 Biberach (DE); Nicola, Thomas, 55218, Ingelheim (DE); Duran, Adil, 88400, Rissegg (DE)
(74) Vertreter: Simon, Elke Anna Maria

(56) Entgegenhaltungen:
- WO-A-02/068420
- WO-A-2004/018468
- WO-A-2004/041820
- WO-A-2005/085246
- JP-A- 2006 045 156
- US-A- 4 005 208
- DONALD W. COMBS, JEFFERY B. PRESS, DENNIS MULVEY, YOLANDA GRAY-NUNEZ, STANLEY G. BELL: "Phosphoryl chloride induced ring contractation of 1,4-benzodiazepinones to chloromethylquinazolines", J. HETEROCYCLIC CHEM., Bd. 23, 1986, Seiten 1263-1264, XP002614645,
- DAVE K G ET AL: "REACTION OF NITRILES UNDER ACIDIC CONDITIONS. PART I. A GENERAL METHOD OF SYNTHESIS OF CONDENSED PYRIMIDINES", JOURNAL OF HETEROCYCLIC CHEMISTRY, WILEY-BLACKWELL PUBLISHING, INC, US, Bd. 17, 1. November 1980 (1980-11-01), Seiten 1497-1500, XP002059630, ISSN: 0022-152X, DOI: DOI:10.1002/JHET.5570170727

## Beschreibung

Die Erfindung betrifft ein verbessertes Verfahren zur Herstellung chiraler 8-(3-Aminopiperidin-1-yl)-xanthine, deren Enantiomere und deren physiologisch verträgliche Salze.

8-(3-Amino-piperidin-1-yl)-xanthine der folgenden allgemeinen Struktur 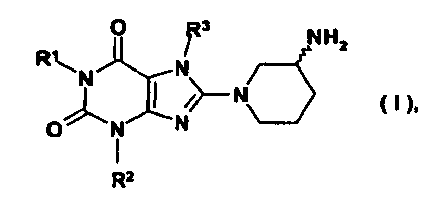 in den R¹ beispielsweise eine gegebenenfalls substituierte Arylmethyl- oder eine gegebenenfalls substituierte Heteroarylmethylgruppe, R² beispielsweise eine Alkylgruppe und R³ beispielsweise eine gegebenenfalls substituierte Benzylgruppe oder eine geradkettige oder verzweigte Alkenyl- oder Alkinylgruppe bedeuten, sind bereits aus den internationalen Anmeldungen WO 02/068420, WO 04/018468, WO 04/018467, WO2004/041820 und WO 2004/046148 bekannt, in denen Verbindungen mit wertvollen pharmakologischen Eigenschaften beschrieben werden, zu denen insbesondere eine Hemmwirkung auf die Aktivität des Enzyms Dipeptidylpeptidase-IV (DPP-IV), gehören. Daher sind Verbindungen diese Typs zur Prävention oder Behandlung von Krankheiten oder Zuständen, die in Zusammenhang mit einer erhöhten DPP-IV Aktivität stehen oder die durch Reduktion der DPP-IV Aktivität verhindert oder gemildert werden können, insbesondere von Diabetes mellitus Typ I oder Typ II, Prädiabetes, oder Verminderung der Glukosetoleranz geeignet.

In der WO 04/018468,wird ein Herstellverfahren offenbart, bei dem 8-(3-Aminopiperidin-1-yl)-xanthine durch Entschützung eines entsprechenden tert.-Butyloxycarbonyl-geschützten Derivats der allgemeinen Formel (II) hergestellt werden. 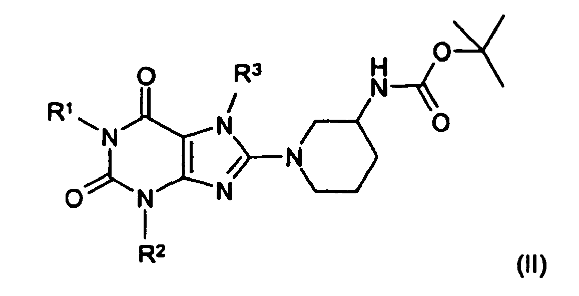 Bei diesem Verfahren traten insbesondere im technischen Maßstab schwer abtrennbare Verunreinigungen auf, welche auf die verwendete Schutzgruppe zurückzuführen sind. Daher war das Verfahren für die technische Herstellung von 8-(3-Aminopiperidin-1-yl)-xanthinen, insbesondere für die Arzneimittelherstellung mit ihren strengen Anforderungen an die Reinheit, nicht geeignet. Des weiteren hatte die Methode den Nachteil, dass die Herstellung des enantiomerenreinen Vorläufers 3-(tert.-Butyloxycarbonylamino)-piperidins aufwendig und teuer ist. Enantiomerenreine Wirkstoffe sind jedoch wegen des Risikos von Nebenwirkungen und zur Reduzierung der Dosis auf ein Minimum für die pharmazeutische Anwendung vorzuziehen. Diese Umstände widersprechen der Eignung des bekannten Verfahrens für die technische Herstellung enantiomerenreiner 8-(3-Amino-piperidin-1-yl)-xanthine.

Im Lichte der oben beschriebenen Nachteile des bekannten Herstellverfahrens ist es die Aufgabe der vorliegenden Erfindung, ein Verfahren bereitzustellen, das die Herstellung enantiomerenreiner 8-(3-Amino-piperidin-1-yl)-xanthine unter Verwendung leicht zugänglicher Ausgangsstoffe in hoher chemischer und optischer Reinheit und ohne großen technischen Aufwand erlaubt. Dieses neue Verfahren soll auch für die Synthese im technischen Maßstab und damit für die kommerzielle Anwendung geeignet sein.

Diese Aufgabe wird durch das erfindungsgemäße Verfahren zur Herstellung chiraler 8-(3-Amino-piperidin-1-yl)-xanthine gelöst. Neben der technischen Durchführbarkeit in hohen Ausbeuten sind sehr gute chemische und optische Reinheiten weitere Vorteile des erfindungsgemäßen Syntheseweges.

Gemäß des erfindungsgemäßen Verfahrens wird der entsprechende Xanthin-Vorläufer (III) mit enantiomerenreinem oder racemischem 3-(Phthalimido)piperidin in geeigneten Lösungsmitteln beim Temperaturen von 20 bis 160°C; vorzugsweise von 80 bis 140°C gemäß Schema 1 umgesetzt. Als Lösungsmittel können beispielsweise Tetrahydrofuran (THF), Dioxan, N,N-Dimethylformamid (DMF), Dimethylacetamid (DMA), N-Methyl-2-pyrrolidon (NMP) oder Dimethylsulfoxid (DMSO) verwendet werden. Vorzugsweise wird NMP verwendet. Anschließend wird die Phthalylschutzgruppe nach an sich bekannten Verfahren abgespalten. Mögliche Abspaltungsmethoden werden z.B. von T.W. Greene in "Protective Groups in Organic Synthesis, Wiley 1981 auf Seite 265 beschrieben (z. B. Hydrazin in Ethanol). 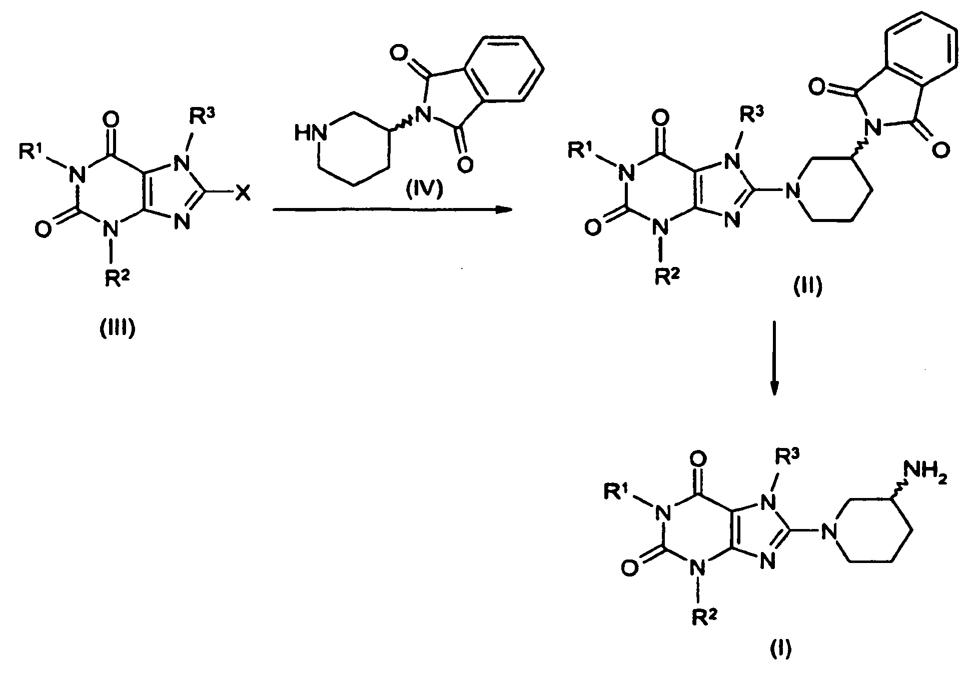 In den obenstehenden Formeln bedeuten

X eine Fluchtgruppe ausgewählt aus der Gruppe der Halogene wie beispielsweise ein Fluor-, Chlor- oder Bromatom oder der Sulfonsäureester wie beispielsweise eine Phenylsulfonyloxy-, p-Toluolsulfonyloxy-, Methylsulfonyloxy- oder Trifluormethylsulfonyloxygruppe, R¹ eine Phenylcarbonylmethyl-, Benzyl-, Naphthylmethyl-, Pyridinylmethyl-, Pyrimidinylmethyl-, Chinolinylmethyl-, Isochinolinylmethyl-, Chinazolinylmethyl-, Chinoxalinylmethyl-, Naphthyridinylmethyl- oder Phenanthridinylmethyl-Gruppe, in der jeweils der aromatische bzw. heteroaromatische Teil durch Rₐ mono- oder disubstituiert ist, wobei die Substituenten gleich oder verschieden sein können und
Rₐ ein Wasserstoff-, Fluor-, Chlor- oder Bromatom oder eine Cyan-,
Methyl-, Trifluormethyl-, Ethyl-, Phenyl-, Methoxy-, Difluormethoxy-,
Trifluormethoxy- oder Ethoxy-Gruppe darstellt,
oder zwei Reste Rₐ, sofern sie an benachbarte Kohlenstoffatome
gebunden sind, auch eine -O-CH₂-O- oder -O-CH₂-CH₂-O- Gruppe
darstellen können,
R² eine Methyl-, Ethyl-, Propyl-, Isopropyl-, Cyclopropyl- oder Phenyl-Gruppe und
R³ eine 2-Buten-1-yl-, 3-Methyl-2-buten-1-yl-, 2-Butin-1-yl-, 2-Fluorbenzyl-, 2-Chlorbenzyl-, 2-Brombenzyl-, 2-lodbenzyl-, 2-Methylbenzyl-, 2-(Trifluormethyl)benzyl- oder 2-Cyanbenzyl-Gruppe bedeuten.

Bevorzugt ist das Verfahren für diejenigen Verbindungen, in denen X ein Chlor- oder Bromatom,
R¹ eine Phenylcarbonylmethyl-, Benzyl-, Naphthylmethyl-, Pyridinylmethyl-, Pyrimidinylmethyl-, Chinolinylmethyl-, Isochinolinylmethyl-, Chinazolinylmethyl-, Chinoxalinylmethyl- oder Naphthyridinylmethyl-Gruppe, in der jeweils der aromatische bzw. heteroaromatische Teil durch Rₐ mono- oder disubstituiert ist, wobei die Substituenten gleich oder verschieden sein können und
Rₐ ein Wasserstoff-, Fluor- oder Chloratom oder eine Cyan-, Methyl-, Ethyl-,
Methoxy- oder Ethoxy-Gruppe darstellt,
R² eine Methyl-, Ethyl-, Propyl-, Isopropyl-, Cyclopropyl- oder Phenyl-Gruppe und
R³ eine 2-Buten-1-yl-, 3-Methyl-2-buten-1-yl-, 2-Butin-1-yl-, 2-Fluorbenzyl-,2-Chlorbenzyl-, 2-Brombenzyl-, 2-lodbenzyl-, 2-Methylbenzyl-, 2-(Trifluormethyl)benzyl-, oder 2-Cyanbenzyl-Gruppe bedeuten.

Besonders bevorzugt ist das Verfahren für diejenigen Verbindungen, in denen X ein Chlor- oder Bromatom,
R¹ eine Cyanbenzyl-, (Cyanpyridinyl)methyl-, Chinolinylmethyl-, (Methylchinolinyl)-methyl-, Isochinolinylmethyl-, (Methylisochinolinyl)methyl-, Chinazolinylmethyl-, (Methylchinazolinyl)methyl-, Chinoxazinylmethyl-, (Methylchinoxalinyl)methyl-, (Dimethychinoxalinyl)methyl- oder Naphthyridinylmethyl-Gruppe,
R² eine Methyl-, Cyclopropyl- oder Phenylgruppe und
R³ eine 2-Buten-1-yl-, 3-Methyl-2-buten-1-yl-, 2-Butin-1-yl-, 2-Chlorbenzyl-, 2-Brombenzyl- oder 2-Cyanbenzyl-Gruppe bedeuten,
insbesondere jedoch für die Verbindungen 1-[(4-Methyl-chinazolin-2-yl)methyl]-3-methyl-7-(2-butin-1-yl)-8-(3-(R)-amino-piperidin-1-yl)-xanthin, 1-[(3-Methyl-isochinolin-1-yl)methyl]-3-methyl-7-(2-butin-1-yl)-8-((R)-3-amino-piperidin-1-yl)-xanthin und 1-[(3-Cyano-pyridin-2-yl)methyl]-3-methyl-7-(2-butin-1-yl)-8-(3-(R)-aminopiperidin-1-yl)-xanthin, wobei X Brom bedeutet.

Bevorzugt wird jeweils (R)-3-(Phthalimido)piperidin als Reagenz verwendet. Die Herstellung der Verbindungen der Formel (III) ist in der bereits oben zitierten Literatur beschrieben und erfolgt nach an sich bekannten Verfahren.

Ein weiterer Erfindungsgegenstand ist ein Verfahren zur Herstellung von optisch aktivem 3-(Phthalimido)piperidin. Dabei wird 3-Aminopyridin zunächst mittels an sich bekannten Verfahren hydriert. Das so erhaltene racemische 3-Aminopiperidin wird dann mittels Phthalsäureanhydrid in das entsprechende Phthalimid überführt. Aus der Lösung des racemischen, rohen Phthalimids (IV) kann mittels D-Weinsäure selektiv das (R)-Enantiomer ausgefällt werden. Aus der Mutterlauge dieser Salzfällung kann ferner durch Zugabe von L-Weinsäure das (S)-Enantiomer von (IV) auf einfache Weise gewonnen werden, und zwar ohne vorherige Abtrennung des noch in der Mutterlauge enthaltenen Überschusses an D-Weinsäure.

Diese extrem einfache Enantiomerentrennung der Verbindung der Formel (IV) ist für den Fachmann überraschend. Die racemische Base aus der Hydrierreaktion muß dazu zuvor nicht aufgereinigt werden. Das Verfahren funktioniert selbst im technischen Maßstab problemlos.

Zudem ist schon die unerwartet saubere Umsetzung von 3-Aminopiperidin mit Phthalsäureanhydrid per se überraschend, da laut Literatur (z.B. US-Patent US 4,005,208, insbesondere Beispiel 27) Gemische zu erwarten sind, die neben dem gewünschten Produkt Derivate, in denen das Ring-Stickstoffatom acyliert ist, enthalten.

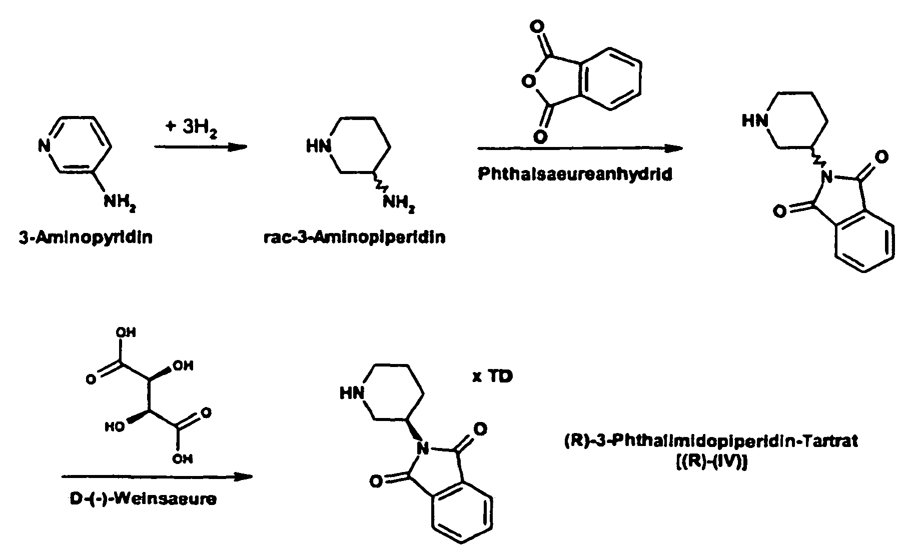

Die folgenden Beispiele sollen die Erfindung näher erläutern: **Beispiel 1**

### D-Weinsäuresalz des R-Enantiomers von 3-(Phthalimido)piperidin

### a. Hydrierung:

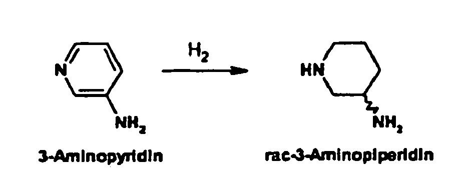

10,00 kg (106,25 mol) 3-Aminopyridin, 500 g Aktivkohle techn. und 65 Liter Essigsäure werden im Hydrierreaktor vorgelegt. 50 g Nishimura Katalysator (ein kommerziell erhältlicher Rhodium/Platin Mischkatalysator) werden in 2,5 Liter Essigsäure aufgeschlemmt zugeben und mit 2,5 Liter Essigsäure nachgespült. Es wird bei 50°C und 100 bar Wasserstoffüberdruck bis zum Stillstand der Wasserstoffaufnahme hydriert und anschließend 30 Minuten bei 50°C nachhydriert. Der Katalysator und die Aktivkohle werden abfiltriert und mit 10 Liter Essigsäure nachwaschen. Die Produktlösung wird ohne Reinigung weiter umgesetzt.

Die Reaktion gelingt auch unter weniger drastischen Drücken.

### b. Acylierung:

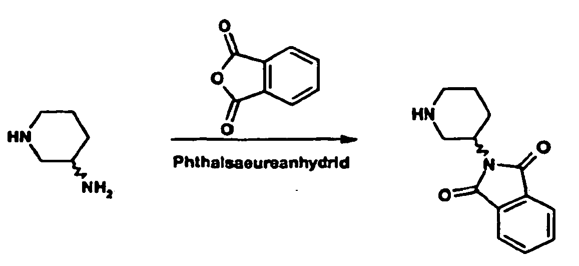

15,74 kg (106,25 mol) Phthalsäureanhydrid werden im Reaktor vorgelegt und mit dem Filtrat aus der Hydrierung versetzt. Es wird mit 7,5 Liter Essigsäure nachgespült, und anschließend wird die Reaktionsmischung zum Rückfluß erhitzt, wobei innerhalb einer Stunde ca. 30% der eingesetzten Essigsäure abdestilliert werden. Die Reaktionslösung wird auf 90°C abgekühlt. Die Produktlösung wird ohne Reinigung weiter umgesetzt.

### c. Racematspaltung:

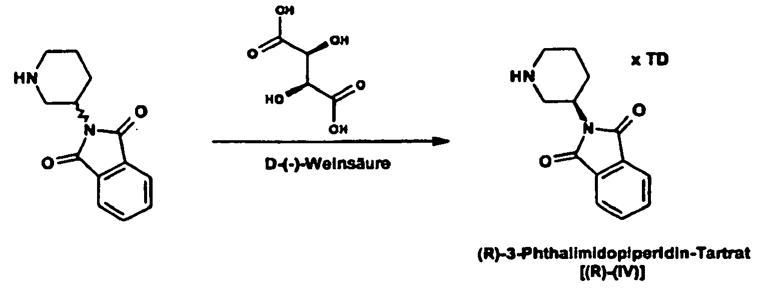

Eine auf 50°C erwärmte Lösung von 11,16 kg D-(-)-Weinsäure (74,38 mol) in 50 Liter absolutem Ethanol wird bei 90°C zur Acylierungsreaktionslösung zudosiert. Es wird mit 10 Liter Ethanol absolut nachgespült und 30 Minuten bei 90°C nachgerührt, wobei das Produkt kristallisiert. Nach dem Abkühlen auf 5°C wird das Produkt abzentrifugiert und mit Ethanol absolut gewaschen. Die Produktlösung wird ohne Reinigung weiter umgesetzt.

### d. Umkristallisation:

Das feuchte Rohprodukt wird in einer Mischung von 50 Liter Aceton und 90 Liter Wasser so lange zum Rückfluß erhitzt, bis eine Lösung entstanden ist. Anschliessend wird auf 5°C abgekühlt, wobei das Produkt auskristallisiert. Die Suspension wird bei 5°C 30 Minuten nachgerührt, das Produkt wird abzentrifugiert und zuletzt mit einer Mischung aus 20 Liter Aceton und 10 Liter Wasser gewaschen. Es wird im Trockenschrank unter Inertisierung bei 45°C getrocknet. Ausbeuten: 11,7 - 12,5 kg (29 - 31 % d. Theorie)

### Beispiel 2

### Synthese von 1-[(4-Methyl-chinazolin-2-yl)methyl]-3-methyl-7-(2-butin-1-yl)-8-(3-(R)-amino-piperidin-1-yl)-xanthin

### a. 2-Chlormethyl-4-methyl-chinazolin

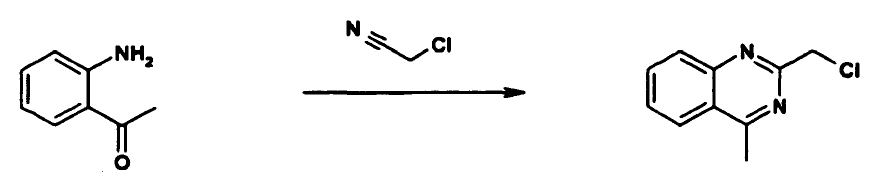

10,00 kg (73,98 mol) 2-Aminoacetophenon werden vorgelegt und 24,5 Liter 1,4-Dioxan zugegeben. Die auf 10°C abgekühlte Lösung wird durch Überleiten mit 16,72 kg (458,68 mol) Chlorwasserstoff versetzt. Die Reaktionsmischung erwärmt sich auf 22 - 25°C. Bei dieser Temperatur wird weiter Chlorwasserstoff übergeleitet. Ab etwa der Hälfte der gesamten Überleitungsmenge wird auf -10°C abgekühlt und weiter übergeleitet. Anschließend wird die entstandene Suspension bei - 10°C über Nacht stehen gelassen.

Eine Lösung aus 6,70 kg (88,78 mol) Chloracetonitril in 2,5 Liter 1,4-Dioxan wird innerhalb von einer Stunde bei -10°C zugegeben. Das Zulaufgefäß wird mit 2 Liter 1,4-Dioxan nachgespült. Danach wird der Reaktorinhalt auf 6°C erwärmt und ca. 2 Stunden nachgerührt.

In einem weiteren Reaktor wird eine Mischung aus 122 Liter Wasser und 62,04 kg (775,31 mol) Natriumhydroxid-Lösung (50 %) vorgelegt und auf 6°C abgekühlt. Das Reaktionsgemisch aus dem ersten Reaktor wird portionsweise zugegeben. Die Innentemperatur beträgt dabei maximal 11 °C. Anschließend wird der erste Reaktor zuerst mit 6 Liter 1,4-Dioxan und dann mit 6 Liter Wasser nachgespült. Die entstandene Suspension wird noch 30 Minuten bei 5°C nachgerührt. Das Produkt wird abzentrifugiert, mit 41 Liter Wasser gewaschen und unter Inertisierung im Trockenschrank bei 35°C getrocknet.

Ausbeute: 10,5 - 12,1 kg (74 - 85 % d. Theorie)

### b. 1-[(4-Methyl-chinazolin-2-yl)methyl]-3-methyl-7-(2-butin-1-yl)-8-brom-xanthin

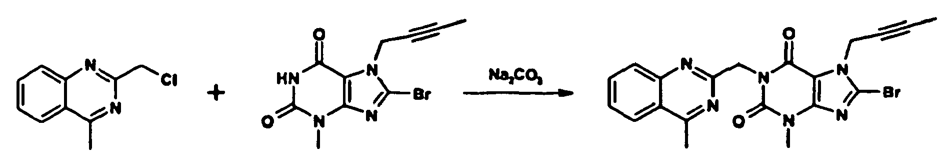

10,00 kg (33,66 mol) 3-Methyl-7-(2-butin-1-yl)-8-brom-xanthin, 7,13 kg (37,02 mol) 2-Chlormethyl-4-methyl-chinazolin, 3,92 kg (37,02 mol) Natriumcarbonat wasserfrei und 30 Liter N-Methyl-2-pyrrolidon werden im Reaktor vorgelegt. Der Reaktorinhalt wird auf 140°C erhitzt und 2 Stunden bei 140°C gerührt. Nach beendeter Reaktion wird die Reaktionsmischung auf 80°C abgekühlt und mit 60 Liter Ethanol 96% sowie anschließend bei 70°C mit 55 Liter Wasser verdünnt. Bei 60°C werden 4,04 kg (67,32 mol) Essigsäure zudosiert und mit 5 Liter Wasser nachgespült. Die entstandene Suspension wird 30 Minuten bei 60°C gerührt, dann auf 23°C abgekühlt und 30 Minuten nachgerührt. Anschließend wird das Produkt abzentrifugiert und zuerst mit einer Mischung von 20 Liter Ethanol 96 % und 20 Liter Wasser, anschließend mit 40 Liter Ethanol 96% und 40 Liter Wasser gewaschen. Es wird im Trockenschrank unter Inertisierung bei 45°C getrocknet.

Ausbeute: 11,6 - 12,6 kg (76 - 83 % d. Theorie)

### c. 1-[(4-Methyl-chinazolin-2-yl)methyl]-3-methyl-7-(2-butin-1-yl)-8-(3-(R)-phthalimido-piperidin-1-yl)-xanthin

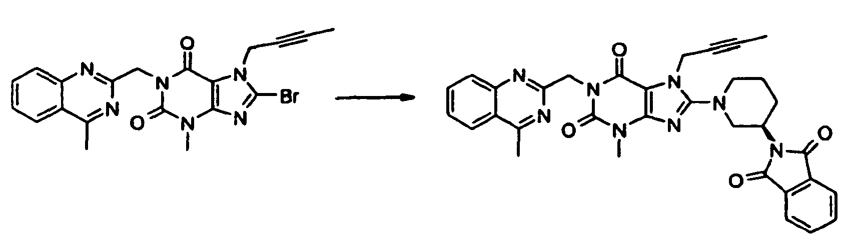

10,00 kg (22,06 mol) 1-[(4-Methyl-chinazolin-2-yl)methyl]-3-methyl-7-(2-butin-1-yl)-8-brom-xanthin, 12,59 kg (33,09 mol) 3-(Phthalimido)piperidin D-Tartrat und 17,5 Liter N-Methyl-2-pyrrolidon werden im Reaktor vorgelegt. Der Reaktorinhalt wird auf 140°C erhitzt. Nach Erreichen der Temperatur werden innerhalb von 20 Minuten 11,41 kg (88,24 mol) Diisopropylethylamin zudosiert. Das Zulaufgefäß wird mit 2,5 Liter N-Methyl-2-pyrrolidon nachgespült und die Reaktionsmischung anschließend für 2 Stunden bei 140°C gerührt. Nach beendeter Reaktion wird die Reaktionsmischung auf 60°C abgekühlt und mit 80 Liter Methanol verdünnt. Die entstandene Suspension wird 30 Minuten bei 50°C gerührt, dann auf 23°C abgekühlt und 30 Minuten nachgerührt. Anschließend wird das Produkt abzentrifugiert und 3 mal mit je 20 Liter Methanol gewaschen. Es wird im Trockenschrank unter Inertisierung bei 45°C getrocknet.

Ausbeute: 12,0 - 12,5 kg (90 - 94 % d. Th.)

### d.1-[(4-Methyl-chinazolin-2-yl)methyl]-3-methyl-7-(2-butin-1-yl)-8-(3-(R)-aminopiperidin-1-yl)-xanthin

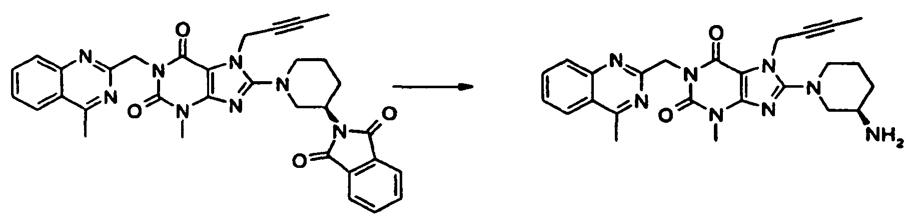

1800 g (3 mol) 1-[(4-Methyl-chinazolin-2-yl)methyl]-3-methyl-7-(2-butin-1-yl)-8-(3-(*R*)-phthalimido-piperidin-1-yl)-xanthin werden in 18 Liter Toluol auf 80-85°C erhitzt. Anschließend werden bei 75-80°C 1,815 Liter (30 mol) Ethanolamin zur Suspension zugeben. Zur Vervollständigung der Reaktion wird 2 Stunden bei 80-85°C nachgerührt, wobei die Feststoffe in Lösung gehen. Anschließend werden die Phasen getrennt. Die Ethanolamin-Phase wird zweimal mit warmem Toluol (je 4 Liter) gewaschen. Die vereinigten Toluol-Phasen werden zweimal mit je 8 Liter 75-80°C warmem Wasser gewaschen. Von der Toluol-Phase werden unter Vakuum 22 Liter Toluol abdestilliert. Zur entstandenen Suspension wird bei 40-50°C 4 Liter tert.-Butylmethylether zudosiert und anschließend auf 0-5°C abgekühlt. Das Produkt wird durch Filtration isoliert, mit tert.-Butylmethylether nachgewaschen und trockengesaugt. Die feuchte Rohsubstanz wird anschließend mit der 5-fachen Menge an absolutem Ethanol zum Rückfluß erhitzt und die heiße Lösung über Aktiv-Kohle klarfiltriert. Nach Abkühlen des Filtrates auf 20°C und Einsetzen der Kristallisation wird mit tert.-Butylmethylether auf das doppelte Volumen verdünnt. Die Suspension wird auf 2°C abgekühlt, 2 Stunden nachgerührt, abgesaugt und im Vakuumtrockenschrank bei 45°C getrocknet.

Ausbeute: 1174 g (83,2% d. Theorie)

### Alternatives Verfahren für Schritt d:

1400 g (2,32 mol) 1-[(4-Methyl-chinazolin-2-yl)methyl]-3-methyl-7-(2-butin-1-yl)-8-(3-(R)-phthalimido-piperidin-1-yl)-xanthin werden in 4,9 I Tetrahydrofuran vorgelegt und anschließend auf 55-65°C erhitzt. Anschließend werden 350 ml Wasser sowie 1433 g (2,32 mol) Ethanolamin zur Suspension zugeben. Zur Vervollständigung der Reaktion wird 3 Stunden bei 60-63°C nachgerührt. Anschließend werden 619 ml 45-%ige Natronlauge sowie 3,85 I Wasser zugesetzt und für 30 min bei 55-65°C gerührt. Zum Reaktionsgemisch werden dann 5,6 I Toluol gegeben, 15 min gerührt und anschließend die Phasen getrennt. Die organische Phase wird mit 2,8 I Wasser bei 55-65°C gewaschen und anschließend abgetrennt. Von der organischen Phase werden unter Vakuum 4,2 l abdestilliert. Anschließend wird bei 65-75°C 1,4 l Methylcyclohexan zugegeben, wobei das Produkt kristallisiert. Die Suspension wird 8-16 h bei 15-25°C gerührt und anschließend auf 0-5°C abgekühlt. Das Produkt wird durch Filtration isoliert, mit 4,2 l Methylcyclohexan nachgewaschen und trockengesaugt und im Vakuum bei 35°C getrocknet. Die getrocknete Rohsubstanz (991 g) wird anschließend mit der 5-fachen Menge an Methanol zum Rückfluß erhitzt, Aktiv-Kohle zugesetzt und filtriert. Das Filtrat wird durch Abdestillieren von Methanol auf ein Volumen von 1,5 I reduziert. Nach Abkühlen des Filtrates auf 45-55°C° wird mit tert.-Butylmethylether auf das vierfache Volumen verdünnt. Die Suspension wird auf 0-5°C abgekühlt, 2 Stunden nachgerührt, abgesaugt, mit tert.-Butylmethylether nachgewaschen und im Vakuumtrockenschrank bei 35°C getrocknet.

Ausbeute: 899 g (81,9 % d. Theorie)

### Beispiel 3

1-[(3-Cyano-pyridin-2-yl)methyl]-3-methyl-7-(2-butin-1-yl)-8-(3-(*R*)-amino-piperidin-1-yl)-xanthin

### a. 3-Cyano-2-(chlormethyl)-pyridin

165,5 g (0,98 mol) 2-Hydroxymethyl-3-pyridincarboxamid werden zusammen mit 270 ml Phosphoroxychlorid für 1 Stunde auf 90-100°C erhitzt. Das Reaktionsgemisch wird auf Raumtemperatur abgekühlt und anschließend in ca. 800 ml 50-60°C warmes Wasser eingetropft. Nach Hydrolyse des Phosphoroxychlorids wird unter Kühlung mit Natronlauge neutralisiert, wobei das Produkt ausfällt. Es wird abfiltriert, mit 300 ml Wasser gewaschen und anschließend bei 35-40°C getrocknet.

Ausbeute: 122,6 g (82% d. Th.)

### Variante zu Verfahrensschritt a: 3-Cyano-2-(chlormethyl)-pyridin

20,0 g (131,45 mmol) 2-Hydroxymethyl-3-pyridincarboxamid werden in 110 ml Acetonitril suspendiert und auf 78°C erwärmt. Innerhalb von 15 Minuten werden 60,65 g (395,52 mmol) Phosphoroxychlorid zudosiert und 2 Stunden auf 81 °C erwärmt. Nach abkühlen auf 22°C wird die Reaktionsmischung in 200 ml 40°C warmes Wasser eingerührt. Nach Zugabe von 100 ml Toluol wird unter Kühlung mit Natronlauge neutralisiert. Nach Phasentrennung wird die organische Phase mit 100 ml Wasser gewaschen. Abtrennung der organische Phase und verdampfen des Lösemittels im Vakuum ergibt zunächst einen öligen Rückstand welcher beim Stehen kristallisiert.

Ausbeute: 16,66 g (83% d.Th)

### b. 1-[(3-Cyano-pyridin-2-yl)methyl]-3-methyl-7-(2-butin-1-yl)-8-brom-xanthin

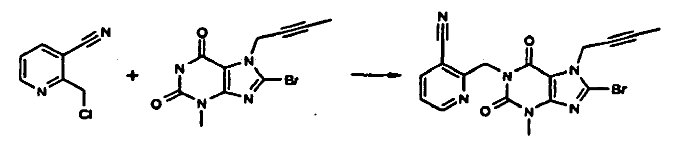

202 g (0,68 mol) 3-Methyl-7-(2-butin-1-yl)-8-brom-xanthin 188,5 g (1,36 mol) Kaliumcarbonat wasserfrei und 1,68 Liter N-Methyl-2-pyrrolidon werden im Reaktor vorgelegt und auf 70°C erwärmt. Anschließend werden 119 g (0,75 mol) 2-Chlormethyl-3-cyano-pyridin in 240 ml N-Methyl-2-pyrrolidin (NMP) zugetropft. Der Reaktorinnalt wird für 19 Stunden bei 70°C gerührt. Nach beendeter Reaktion wird der Reaktionsmischung 2,8 Liter Wasser zugesetzt und auf 25°C abgekühlt. Das Produkt wird abfiltriert, mit 2 Liter Wasser gewaschen und im Trockenschrank unter Inertisierung bei 70°C getrocknet.

Ausbeute: 257,5 g (91% d. Th.)

### c. 1-[(3-Cyano-pyridin-2-yl)methyl]-3-methyl-7-(2-butin-1-yl)-8-(3-(R)-phthalimido-piperidin-1-yl)-xanthin

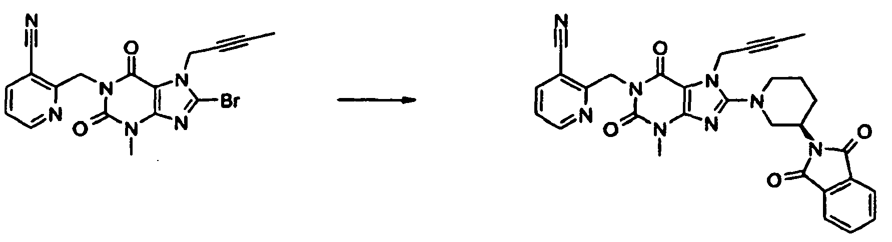

230 g (0,557 mol) 1-[(3-Cyano-pyridin-2-yl)methyl]-3-methyl-7-(2-butin-1-yl)-8-brom-xanthin, 318 g (0,835 mol) 3-(Phthalimido)piperidin D-Tartrat und 1,15 Liter N-Methyl-2-pyrrolidon werden im Reaktor vorgelegt. Der Reaktorinhalt wird auf 140°C erhitzt. Nach Erreichen der Temperatur werden innerhalb von 20 Minuten 478 ml (2,78 mol) Diisopropylethylamin zudosiert und die Reaktionsmischung anschließend für 2 Stunden bei 140°C gerührt. Anschließend wird die Reaktionsmischung auf 75°C abgekühlt und mit 720 ml Methanol verdünnt. Danach werden bei 68-60°C 2,7 Liter Wasser zugegeben und auf 25°C abgekühlt. Das Produkt wird abfiltriert und mit 2 Liter Wasser gewaschen. Es wird im Trockenschrank unter Inertisierung bei 70°C getrocknet.

Das so erhaltene Rohprodukt wird anschließend in 1 Liter Methanol in der Siedehitze verrührt, heiß filtriert mit 200 ml Methanol gewaschen und anschließend bei 70°C unter Inertisierung getrocknet.

Ausbeute: 275 g (88 % d. Th.)

### d. 31-[(3-Cyano-pyridin-2-yl)methyl]-3-methyl-7-(2-butin-1-yl)-8-(3-(R)-amino-piperidin-1-yl)-xanthin

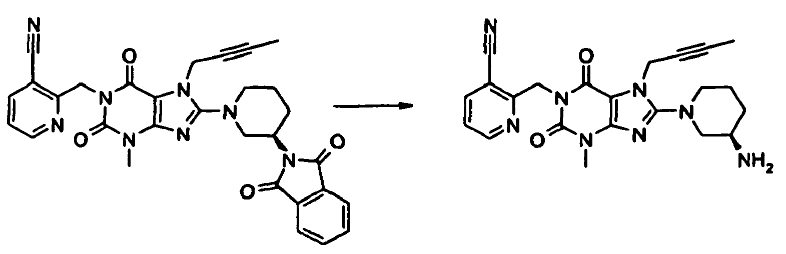

412,5 g (0,733 mol) 1-[(3-Cyano-pyridin-2-yl)methyl]-3-methyl-7-(2-butin-1-yl)-8-(3-(R)-phthalimido-piperidin-1-yl)-xanthin werden in 4125 ml Toluol auf 80°C erhitzt. Anschließend werden bei 75-80°C 445 ml Ethanolamin (7,33 mol) zur Suspension zugeben. Zur Vervollständigung der Reaktion wird 2 Stunden bei 80-85°C nachgerührt, wobei die Feststoffe in Lösung gehen. Anschließend werden die Phasen getrennt. Die Ethanolamin-Phase wird zweimal mit warmen Toluol (je 1 Liter) extrahiert. Die vereinigten Toluol-Phasen werden zweimal mit je 2 Liter 75-80°C warmen Wasser gewaschen. Die Toluol-Phasen werden mit Natriumsulfat getrocknet, filtriert und anschließend durch Destillation im Vakuum auf ein Volumen von ca. 430 ml reduziert. Anschließend wird bei 50-55°C 1 Liter tert.-Butylmethylether zudosiert und danach auf 0-5°C abgekühlt. Das Produkt wird durch Filtration isoliert, mit tert.-Butylmethylether nachgewaschen und im Trockenschrank bei 60°C getrocknet.

Ausbeute: 273 g (86 % d.Theorie) Schmelzpunkt: 188 ± 3°C

Analog zu den Beispielen 2 und 3 wird auch 1-[(3-Methyl-isochinolin-1-yl)methyl]-3-methyl-7-(2-butin-1-yl)-8-((R)-3-amino-piperidin-1-yl)-xanthin hergestellt.

## Patentansprüche

1. Verfahren zur Herstellung eines Arzneimittels umfassend die Verwendung einer Verbindung der allgemeinen Formel (I) 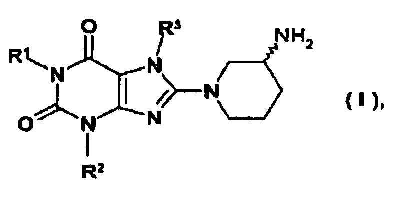 oder eines Enantiomers oder Salzes davon,
in der R¹ eine Phenylcarbonylmethyl-, Benzyl-, Naphthylmethyl-, Pyridinylmethyl-, Pyrimidinylmethyl-, Chinolinylmethyl-, Isochinolinylmethyl-, Chinazolinylmethyl-, Chinoxalinylmethyl-, Naphthyridinylmethyl- oder Phenanthridinylmethyl-Gruppe bedeutet, in der jeweils der aromatische bzw. heteroaromatische Teil durch Rₐ mono- oder disubstituiert ist, wobei die Substituenten gleich oder verschieden sein können und
Rₐ ein Wasserstoff-, Fluor-, Chlor- oder Bromatom oder eine Cyan-, Methyl-, Trifluormethyl-, Ethyl-, Phenyl-, Methoxy- Difluormethoxy-, Trifluormethoxy- oder Ethoxy-Gruppe darstellt,
oder zwei Reste Rₐ, sofern sie an benachbarte Kohlenstoffatome gebunden sind, auch eine -C-CH₂-O- oder -O-CH₂-CH₂-O- Gruppe darstellen können,
R² eine Methyl-, Ethyl-, Propyl-, isopropyl-, Cyclopropyl- oder Phenyl-Gruppe und
R³ eine 2-Buten-1-yl-, 3-Methyl-2-buten-1-yl-, 2-Butin-1-yl-, 2-Fluorbenzyl-, 2-Chlorbenzyl-, 2-Brombenzyl-, 2-Iodbenzyl-, 2-Methylbenzyl-, 2-(Trifluormethyl)-benzyl- oder 2-Cyanbenryl-Gruppe bedeuten,
erhalten nach einem Verfahren umfassend die folgende Syntheseschritte:
a) Umsetzung einer Verbindung der allgemeinen Formel (III) 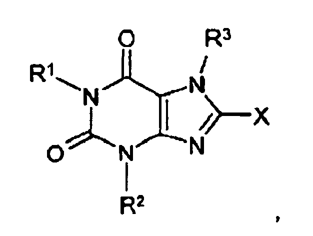 in der X eine Fluchtgruppe ausgewählt aus der Gruppe der Halogene oder der Sulfonsäureester bedeutet und
R¹ bis R³ wie oben erwähnt definiert sind, mit 3-(Phthalimido)piperidin oder einem Enantiomer davon,
b) Entschützung der so erhaltenen Verbindung der allgemeinen Formel (II) 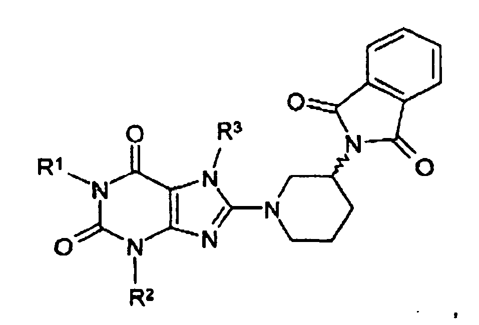 in der R¹ bis R³ wie oben erwähnt definiert sind und
c) gegebenenfalls Überführung in ein physiologisch verträgliches Salz;
und gegebenenfalls eines oder mehrerer inerter Trägerstoffe und/oder Verdünnungsmittel.

2. Verfahren zur Herstellung eines Arzneimittels gemäß Anspruch 1, wobei X ein Bromatom,
R¹ eine (4-Methyl-chinazolin-2-yl)methyl-, (3-Methyl-isochinolin-1-yl)methyl- oder (3-Cyan-pyridin-2-yl)methylgruppe,
R² eine Methylgruppe und
R³ eine 2-Butin-1-ylgruppe bedeuten.

3. Verfahren zur Herstellung eines Arzneimittels gemäß Anspruch 1 oder 2, wobei in Schritt a) (R)-3-(Phthalimido)piperidin als Reaktionspartner verwendet wird.

4. Verfahren zur Herstellung eines Arzneimittels gemäß Anspruch 3, wobei
X ein Bromatom,
R¹ eine (4-Methyl-chinazolin-2-yl)methylgruppe,
R² eine Methylgruppe und
R³ eine 2-Butin-1-ylgruppe bedeuten.

5. Verfahren zur Herstellung eines Arzneimittels gemäß Anspruch 4, ferner umfassend die Kristallisation der Verbindung der Formel (I) aus einer Methanol oder Ethanol Lösung.

6. Verfahren zur Herstellung eines Arzneimittels gemäß Anspruch 5, wobei die Lösung ferner tert.-Butylmethylether enthält.

7. Verfahren zur Herstellung eines Arzneimittels gemäß Anspruch 4, wobei das Verfahren zur Herstellung der Verbindung der Formel (I) anschließend die Kristallisation der Verbindung der Formel (I) aus Methanol aufweist.

8. (R)-3-(Phthalimido)piperidin D-Tartrat, oder (S)-3-(Phthalimido)piperidin L-Tartrat.

9. Verfahren zur Herstellung eines Arzneimittels umfassend die Verwendung einer Verbindung der allgemeinen Formel 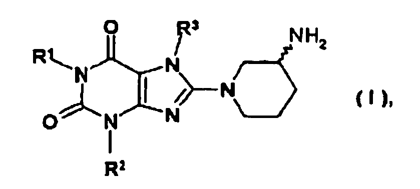 oder eines Enantiomers davon, in dem
R¹ eine (4-Methyl-chinazolin-2-yl)methylgruppe,
R² eine Methylgruppe und
R³ eine 2-Butin-1-yigruppe bedeuten,
erhalten nach dem Verfahren gemäß einem der Ansprüche 4 bis 7;
und gegebenenfalls eines oder mehrerer inerter Trägerstoffe und/oder Verdünnungsmittel.

10. Verfahren zur Herstellung eines Arzneimittels umfassend die Verwendung einer Verbindung gemäß Anspruch 9, erhalten nach dem Verfahren gemäß Anspruch 7, und gegebenenfalls eines oder mehrerer inerter Trägerstoffe und/oder Verdünnungsmittel.

11. Verfahren zur Herstellung eines Arzneimittels gemäß einem der Anspruch 1 bis 3, wobei das Arzneimittel zur Behandlung von Diabetes mellitus Typ I und Typ **II,** Prädiabetes oder Verminderung der Glukosetoleranz, Arthritis, Adipositas, Allograft Transplantation und durch Calcitonin verursachte Osteoporose geeignet ist.

12. Verfahren zur Herstellung eines Arzneimittels umfassend die Verwendung einer Verbindung der allgemeinen Formel **(I)** oder eines Enantiomers davon, in der R¹ bis R³ wie in einem der Ansprüche 1 bis 2 definiert sind, erhalten nach einem Verfahren umfassend die Entschützung einer Verbindung der allgemeinen Formel (II) wie in Anspruch 1 gezeigt, in der R¹ bis R³ wie in einem der Ansprüche 1 bis 2 definiert sind, oder eines Enantiomers davon;
und gegebenenfalls eines oder mehrerer inerter Trägerstoffe und/oder Verdünnungsmittel.

13. Verfahren zur Hersteflung eines Arzneimittels umfassend die Verwendung einer Verbindung der folgenden Formel (I) 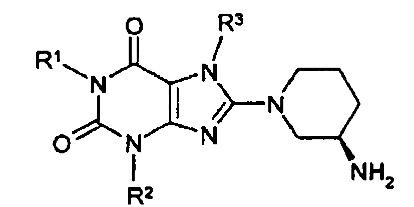 wobei
R¹ eine (4-Methyl-chinazolin-2-yl)methylgruppe,
R² eine Methylgruppe und
R³ eine 2-Butin-1-ylgruppe bedeuten,
erhalten nach einem Verfahren umfassend die Entschützung einer Verbindung der folgenden Formel (II) 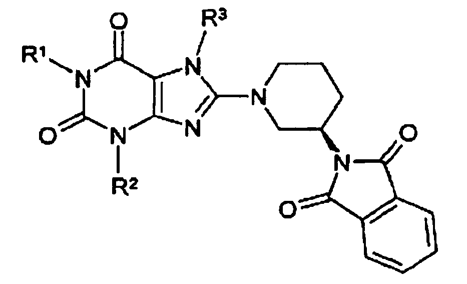
wobei
R¹ eine (4-Methyl-chinazolin-2-yl)methylgruppe,
R² eine Methylgruppe und
R³ eine 2-Butin-1-ylgruppe bedeuten;
und gegebenenfalls eines oder mehrerer inerter Trägerstoffe und/oder Verdünnungsmittel.

14. Verfahren zur Herstellung eines Arzneimittels gemäß Anspruch 13, ferner umfassend die Kristallisation der Verbindung der Formel (I) aus einer Ethanol Lösung.

15. Verfahren zur Herstellung eines Arzneimittels gemäß Anspruch 13 or 14, wobei die Entschatzung der Phthalyl Schutzgruppe der Verbindung der Formel (II) in der Gegenwart von Ethanolamin, vorzugsweise in einer Mischung von Tetrahydrofuran und Wasser oder in Toluol durchgeführt wird.

## Claims

1. Process for preparing a medicament, comprising the use of a compound of general formula (I) 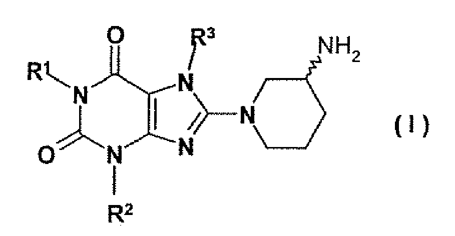 or an enantiomer or salt thereof,
wherein R¹ is a phenylcarbonylmethyl, benzyl, naphthylmethyl, pyridinylmethyl, pyrimidinylmethyl, quinolinylmethyl, isoquinolinylmethyl, quinazolinylmethyl, quinoxalinylmethyl, naphthyridinylmethyl or phenanthridinylmethyl group in which the aromatic or heteroaromatic moiety is in each case mono- or disubstituted by Rₐ, where the substituents may be the same or different and
Rₐ is a hydrogen, fluorine, chlorine or bromine atom or a cyano, methyl, trifluoromethyl, ethyl, phenyl, methoxy, difluoromethoxy, trifluoromethoxy or ethoxy group,
or two Rₐ groups, when they are bonded to adjacent carbon atoms, may also be an -O-CH₂-O- or -O-CH₂-CH₂-O- group,
R² is a methyl, ethyl, propyl, isopropyl, cyclopropyl or phenyl group and
R³ is a 2-buten-1-yl, 3-methyl-2-buten-1-yl, 2-butyn-1-yl, 2-fluorobenzyl, 2-chlorobenzyl, 2-bromobenzyl, 2-iodobenzyl, 2-methylbenzyl, 2-(trifluoromethyl)benzyl or 2-cyanobenzyl group,
obtained by a process comprising the following synthesis steps:
a) reaction of a compound of the general formula (III) 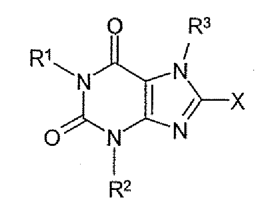 wherein X is a leaving group selected from the group of the halogens or the sulphonic acid esters and
R¹ to R³ are defined as mentioned above, with 3-(phthalimido)piperidine or an enantiomer thereof,
b) deprotection of the compound of general formula (II) thus obtained 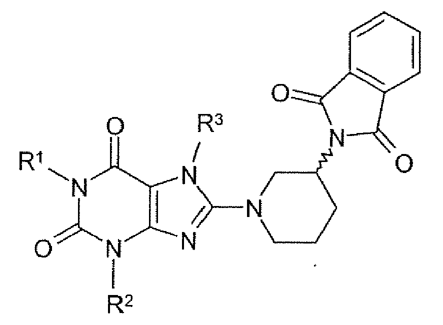 wherein R¹ to R³ are defined as mentioned above and
c) optional conversion to a physiologically acceptable salt;
and optionally one or more inert carriers and/or diluents.

2. Process for preparing a medicament according to claim 1, wherein
X is a bromine atom,
R¹ is a (4-methylquinazolin-2-yl)methyl, (3-methylisoquinolin-1-yl)methyl or (3-cyanopyridin-2-yl)methyl group,
R² is a methyl group and
R³ is a 2-butyn-1-yl group.

3. Process for preparing a medicament according to claim 1 or 2, wherein (R)-3-(phthalimido)piperidine is used as a reactant in step a).

4. Process for preparing a medicament according to claim 3, wherein
X is a bromine atom,
R¹ is a (4-methylquinazolin-2-yl)methyl group,
R² is a methyl group and
R³ is a 2-butyn-1-yl group.

5. Process for preparing a medicament according to claim 4, further comprising the crystallisation of the compound of formula (I) from a methanol or ethanol solution.

6. Process for preparing a medicament according to claim 5, wherein the solution further contains tert.-butylmethyl ether.

7. Process for preparing a medicament according to claim 4, wherein the process for preparing the compound of formula (I) comprises subsequently crystallising the compound of formula (I) from ethanol.

8. (R)-3-(Phthalimido)piperidine D-tartrate or (S)-3-(phthalimido)piperidine L-tartrate.

9. Process for preparing a medicament, comprising the use of a compound of general formula 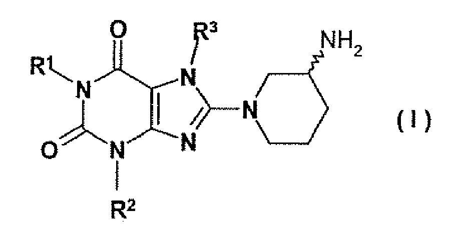 or an enantiomer thereof, wherein
R¹ is a (4-methylquinazolin-2-yl)methyl group,
R² is a methyl group and
R³ is a 2-butyn-1-yl group,
obtained by the process according to one of claims 4 to 7;
and optionally one or more inert carriers and/or diluents.

10. Process for preparing a medicament, comprising the use of a compound according to claim 9, obtained by the process according to claim 7, and optionally one or more inert carriers and/or diluents.

11. Process for preparing a medicament according to one of claims 1 to 3, wherein the medicament is suitable for the treatment of type I and type II diabetes mellitus, prediabetes or reduced glucose tolerance, arthritis, obesity, allograft transplantation and osteoporosis caused by calcitonin.

12. Process for preparing a medicament, comprising the use of a compound of general formula (I) or an enantiomer thereof, wherein R¹ to R³ are defined as in one of claims 1 to 2, obtained by a process comprising deprotecting a compound of general formula (II) as shown in claim 1, wherein R¹ to R³ are defined as in one of claims 1 to 2, or an enantiomer thereof; and optionally one or more inert carriers and/or diluents.

13. Process for preparing a medicament, comprising the use of a compound of the following formula (I) 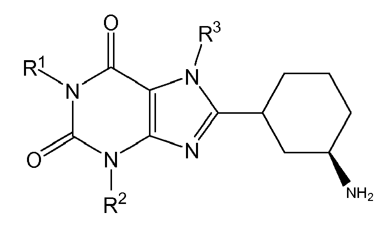 wherein
R¹ is a (4-methylquinazolin-2-yl)methyl group,
R² is a methyl group and
R³ is a 2-butyn-1-yl group,
obtained by a process comprising deprotecting a compound of the following formula (II) 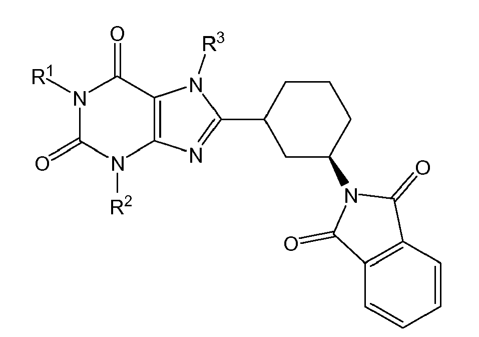 wherein
R¹ is a (4-methylquinazolin-2-yl)methyl group,
R² is a methyl group and
R³ is a 2-butyn-1-yl group,
and optionally one or more inert carriers and/or diluents.

14. Process for preparing a medicament according to claim 13, further comprising crystallising the compound of formula (I) from an ethanol solution.

15. Process for preparing a medicament according to claim 13 or 14, wherein the deprotection of the phthalyl protecting group of the compound of formula (II) is carried out in the presence of ethanolamine, preferably in a mixture of tetrahydrofuran and water or in toluene.

## Revendications

1. Procédé de production d'un médicament comprenant l'utilisation d'un composé de formule générale (I) 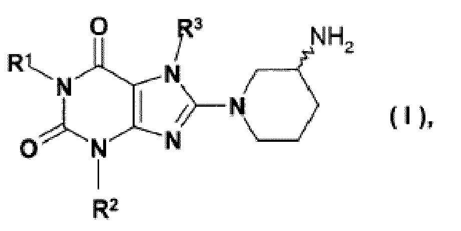 ou d'un énantiomère ou d'un sel de celui-ci,
dans laquelle R¹ représente un groupe phényl-carbonylméthyle, benzyle, naphtylméthyle, pyridinyl-méthyle, pyrimidinylméthyle, quinolinylméthyle, isoquinolinylmétyle, quinazolinylméthyle, quino-xalinylméthyle, naphtyridinylméthyle ou phénanthridinylméthyle, dans lequel respectivement la partie aromatique ou hétéroaromatique est mono-ou disubstituée par Rₐ, les substituants pouvant être identiques ou différents et
Rₐ représente un atome d'hydrogène, de fluor, de chlore ou de brome ou un groupe cyano, méthyle, trifluorométhyle, éthyle, phényle, méthoxydi-fluorométhoxy, trifluorométhoxy ou éthoxy,
ou deux radicaux Rₐ peuvent représenter, dans la mesure où ils sont liés à des atomes de carbone voisins, également, un groupe -O-CH₂-O- ou -O-CH₂-CH₂-O-,
R² représente un groupe méthyle, éthyle, propyle, isopropyle, cyclopropyle ou phényle, et
R³ représente un groupe 2-butén-1-yle, 3-méthyl-2-butén-1-yle, 2-butin-1-yle, 2-fluorobenzyle, 2-chlorobenzyle, 2-bromobenzyle, 2-iodobenzyle, 2-méthylbenzyle, 2-(trifluorométhyl)-benzyle ou 2-cyanobenzyle,
obtenu selon un procédé comprenant les étapes de synthèse suivante :
a) conversion d'un composé de formule générale (III) 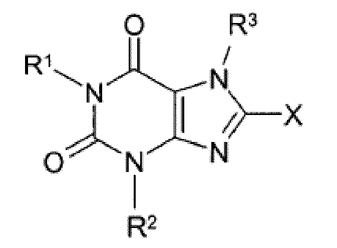 dans laquelle X désigne un groupe partant choisi dans le groupe des halogènes ou de l'ester d'acide sulfonique et
les R¹ à R³ sont tels que définis ci-dessus, avec la 3-(phtalimido)pipéridine ou un énantiomère de celui-ci,
b) déprotection du composé de formule générale (II) ainsi obtenu 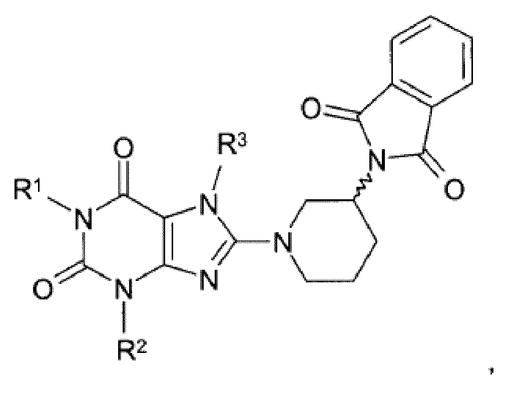 dans laquelle les R¹ à R³ sont tels que définis ci-dessus et
c) éventuellement, conversion en un sel physiologiquement acceptable ;
et éventuellement d'un ou plusieurs véhicules et/ou diluants inertes.

2. Procédé de production d'un médicament selon la revendication 1, dans lequel
X est un atome de brome,
R¹ représente un groupe (4-méthyl-quinazolin-2-yl)méthyle, (3-méthyl-isoquinolin-1-yl)méthyle ou (3-cyano-pyridin-2-yl)méthyle,
R² représente un groupe méthyle et
R³ représente un groupe 2-butin-1-yle.

3. Procédé de production d'un médicament selon la revendication 1 ou 2, dans lequel, à l'étape a), on utilise la (R)-3-(phtalimido)pipéridine comme partenaire réactionnel.

4. Procédé de production d'un médicament selon la revendication 3, dans lequel
X est un atome de brome,
R¹ est un groupe (4-méthylquinazolin-2-yl)méthyle, R² représente un groupe méthyle, et
R³ représente un groupe 2-butin-1-yle.

5. Procédé de production d'un médicament selon la revendication 4, comprenant en outre la cristallisation du composé de formule (I) à partir d'une solution de méthanol ou d'éthanol.

6. Procédé de production d'un médicament selon la revendication 5, dans lequel la solution contient en outre du tert.-butylméthyléther.

7. Procédé de production d'un médicament selon la revendication 4, le procédé de production du composé de formule (I) présentant ensuite la cristallisation du composé de formule (I) à partir d'éthanol.

8. D-tartrate de (R)-3-(phtalimido)pipéridine ou L-tartrate de (S)-3-(phtalimido)pipéridine.

9. Procédé de production d'un médicament comprenant l'utilisation d'un composé de formule générale 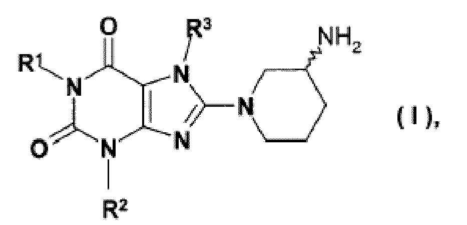 ou d'un énantiomère de celui-ci, dans laquelle R¹ représente un groupe (4-méthyl-quinazolin-2-yl)méthyle,
R² représente un groupe méthyle et
R³ représente un groupe 2-butin-1-yle obtenu selon le procédé selon l'une des revendications 4 à 7 ;
et éventuellement d'un ou plusieurs véhicules et/ou diluants inertes.

10. Procédé de production d'un médicament comprenant l'utilisation d'un composé selon la revendication 9, obtenu selon le procédé selon la revendication 7, et éventuellement d'un ou plusieurs véhicules et/ou diluants inertes.

11. Procédé de production d'un médicament selon l'une des revendications 1 à 3, dans lequel le médicament est approprié pour le traitement du diabète sucré de type I et de type II, du prédiabète ou pour la réduction de la tolérance au glucose, de l'arthrite, de l'adiposité, d'une allogreffe et de l'ostéoporose induite par la calcitonine.

12. Procédé de production d'un médicament, comprenant l'utilisation d'un composé de formule générale (I) ou d'un énantiomère de celui-ci, dans lequel
les R¹ à R³ sont tels que définis dans la revendication 1 à 2, obtenus selon un procédé comprenant la déprotection d'un composé de formule générale (II) comme indiqué dans la revendication 1, dans lequel les R¹ à R³ sont tels que définis dans l'une des revendications 1 à 2, ou d'un énantiomère de celui-ci ;
et éventuellement, d'un ou plusieurs véhicules et/ou diluants inertes.

13. Procédé de production d'un médicament comprenant l'utilisation d'un composé de formule (I) suivante 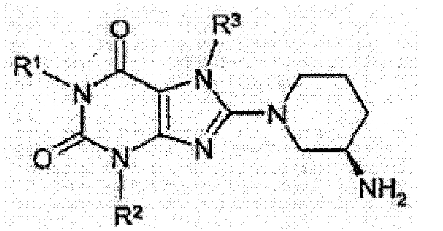 dans laquelle
R¹ représente un groupe (4-méthyl-quinazolin-2-yl)méthyle,
R² représente un groupe méthyle et
R³ représente un groupe 2-butin-1-yle obtenu selon un procédé comprenant la déprotection d'un composé de formule générale (II) 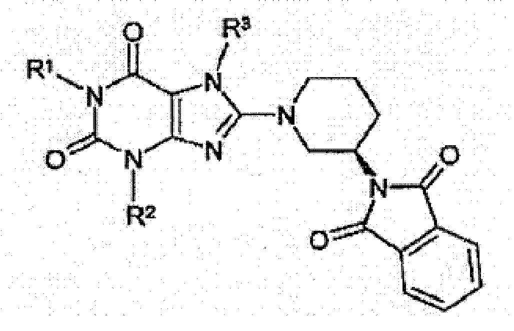
dans laquelle
R¹ représente un groupe (4-méthyl-quinazolin-2-yl)méthyle,
R² représente un groupe méthyle et
R³ représente un groupe 2-butin-1-yle et éventuellement, d'un ou plusieurs véhicules et/ou diluants inertes.

14. Procédé de production d'un médicament selon la revendication 13, comprenant en outre la cristallisation du composé de formule (I) à partir d'une solution d'éthanol.

15. Procédé de production d'un médicament selon la revendication 13 ou 14, dans lequel la déprotection du groupe protecteur phtalyle du composé de formule (II) est réalisée en présence d'éthanolamine, de préférence dans un mélange de tétrahydrofurane et d'eau ou dans du toluène.
